# EUROPEAN PATENT SPECIFICATION

(11) **EP 2 284 742 B1**
(45) Date of publication and mention of the grant of the patent: **29.04.2020**
(21) Application number: 10012526.9
(22) Date of filing: 21.11.2005
(51) Int. Cl.: G16H 20/17

(54) **PATIENT INPUT MONITORING**
Überwachung von Patientendaten
SURVEILLANCE DE DONNEES DE PATIENTES

(30) Priority: 29.11.2004 US 999095
(43) Date of publication of application: 16.02.2011
(62) Divisional of application: 05852118.8
(73) Proprietor: CareFusion 303, Inc., San Diego, CA 92130 (US)
(72) Inventor: Russell, Claudia, J., San Diego CA 92130 (US)
(74) Representative: Epping - Hermann - Fischer

(56) References cited:
- WO-A2-2004/069095
- US-A1- 2001 044 731
- US-A1- 2003 065 308

## Description

### BACKGROUND OF THE INVENTION

The present invention relates generally to medical fluid delivery, and more particularly, to a system and a method for monitoring the total delivery of medical fluids to a patient.

The intravenous infusion of medical fluids into patients is used in the treatment of many different diseases. Depending on the physician, the fluids are delivered to the patient by means of a surgically inserted, main-line catheter or at a peripheral site, such as the patient's arm or leg. Often, due to the patient's condition, it is critical that the prescribed medication dose be administered at the prescribed rates during the designated period of time.

Infusion pumps are often used to administer medications to a patient. The administration may be conducted in small discrete doses or may be given at an essentially continuous rate. Infusion pump therapy may be electronically controlled to deliver precise, metered doses at exactly determined levels, thereby providing a beneficial gradual infusion of medication to the patient. In this manner, the infusion pump is able to mimic the natural process whereby chemical balances are maintained precisely by operating on a continuous time basis.

When a patient undergoes infusion therapy, certain data about the infusion can be acquired and made available to various members of the hospital staff. So that a patient's treatment can be more effectively managed, information related to the medications delivered to a patient should be generated in a relatively short time. However, in many cases, some or all of this data is typically manually recorded on a paper chart that is affixed to the patient's bed. A disadvantage of manual recordation of such data is that it is slow and tedious. Consequently, such data is susceptible to later misinterpretation because of illegibility, incompleteness, and may even be mislocated because of the sheer volume of data being recorded and tracked. Human error contributes greatly to such disadvantages. Thus, it would be desirable to provide for automatic recordation of data related to therapeutic fluid delivery during an infusion.

Information regarding a patient's past infusion therapy may be absolutely essential for later effective and timely treatment. Unfortunately, this essential data may not be easily retrievable from the patient's records. The necessary data may be overlooked for the reasons mentioned above; *i.e.,* it may have been mistranscribed, or entered in an incorrect location on the patient's paper chart, as well as other reasons. In other circumstances, because of the urgency associated with the patient's condition, the medical team may not have time to search through a patient's paper chart or file for certain past therapy data. In any case, failure to easily access such data may result in an inability to obtain the benefit of such prior knowledge concerning past medication delivery when developing or assessing the patient's present and future treatment plan.

In addition, a patient's treatment plan may consist of a series of instructions that may change during the course of treatment. In a manually recorded system, such as a paper chart attached to a patient's bed, it is more difficult to ensure that all details of a treatment plan are recorded; including changes to instructions occurring during treatment. It is also more difficult to track such manually recorded data. Even missing only portions of a patient's prior treatment can give an incomplete picture and lead to a less effective future treatment strategy. As a result, such data may not be available later when further treatment is being assessed.

If the medical team cannot access essential data about a patient in a timely and complete manner, the quality and effectiveness of the care and treatment of the patient may be compromised. Therefore, it would be beneficial if all data obtained from and about a patient in a hospital were immediately accessible to various members of the medical team in accordance with the function performed by those members. There would be a further benefit if those members could select certain data and format such data as desired so that even more effective data usage would result.

Additionally, many hospitals have changed the way in which patients are charged for services. In the past, patients were typically charged on the basis of the number of days hospitalized. With recent changes in health care management and practice, patients are now more likely to be charged on the basis of actual treatments received. Greater efficiency in the treatment of patients is emphasized. Hospitals scrutinize the effect of a treatment on a patient closely, with increased monitoring, observation, and recordation of the patient's responses to treatment. The increased amount of data that must be recorded about a patient makes the existing manual entry, paper chart system extremely cumbersome and time consuming for the medical team. At the same time, accuracy is desirable both for the patient and the healthcare facility for the reasons discussed.

Healthcare facilities are also faced with a competitive environment in which they must constantly remain efficient and yet simultaneously improve patient care. Health care deliverers face increased complexity in the types of treatment and services available, and must also provide these complex treatments and services efficiently. This places a premium on the facility's ability to provide complex treatment while maintaining complete and detailed medical records for each patient. Burdensome recording systems for the administration of medications and usage of supplies involved in a patient's treatment may result in decreased accuracy and thus be undesirable in view of today's more complex treatments.

Examples of a distributed remote asset and medication management drug delivery system appears in US-A-2001/004731 and WO-A-96/28209.

WO 2004/069095 A2 relates to a multi-purpose user interface for a healthcare system having a medical device and a first central computer. The user interface comprises a housing; a processor; a memory; a communications interface for providing communications between the user interface and the first central computer; and a display for displaying a medical prompt and for displaying medical information received from the first central computer.

Hence, those skilled in the art have recognized a need for an improved system and method by which the total patient input of medications can be monitored at any time and can be displayed for immediate consideration. Those skilled in the art have also recognized a need for the ability to format the displayed total patient medication data in various selectable ways depending on the needs of the particular healthcare personnel considering the data. Such a system and method would assist healthcare facilities to provide higher quality patient care. More accurate medication data about the patient would be made available thereby increasing the ability to select more efficient present and future treatment programs. The present invention satisfies these and other needs.

### INVENTION SUMMARY

The invention is defined by the claims. In the following, parts of the description and drawings referring to embodiments which are not covered by the claims are not presented as embodiments of the invention, but as examples useful for understanding the invention.

Briefly and in general terms, the subject application is directed to a monitoring system for monitoring the total delivery of medication to a patient while that patient resides in a healthcare facility. The system is patient-based rather than being confined to a single infusion pump or single infusion pump group. The system accumulates patient data related to the delivery of medication, including infusions of fluids, oral delivery of fluids and pills, and other deliveries of medications, and permits an operator to format that data in various ways for display. Selectable preprogrammed data formats are available or the operator may format the data in other ways as desired by the nurse or operator of the monitoring system.

In accordance with certain aspects of the application, there is provided a system for monitoring medication delivery to a patient while the patient is in a healthcare facility, the monitoring system comprising a memory in which is stored identification data about the patient and medication delivery data about the patient a display on which identification data and medication delivery data about a patient is displayed and a processor that receives patient identification data and medication delivery data about the patient from multiple sources in the healthcare facility while the patient is in the healthcare facility and stores the identification data and medication delivery data in the memory wherein the processor also retrieves the patient identification data and medication delivery data from the memory in response to a request therefor, formats the retrieved data in accordance with a format request, and displays the retrieved and formatted data on the display.

In accordance with more detailed aspects, the system further comprises an infusion pump in which is located the memory, the display, and the processor, and in which the processor receives the medication delivery data from the infusion pump. Further, the processor also compares infusion programming commands directed to the infusion pump to a drug library having predetermined limits and provides an alert if a received infusion programming command is outside of a limit.

In other more detailed aspects, the system further comprises a controller connected to an infusion pump, the memory, the display, and the processor being located in the controller. The processor receives the medication delivery data from the infusion pump. The processor also compares infusion programming commands directed to the infusion pump to a drug library having predetermined limits and provides an alert if a received infusion programming command is outside of a limit.

In further detailed aspects, the memory also stores vital signs data about the patient, the display also displays vital signs data about the patient, and the processor also receives patient vital signs data while the patient is in the healthcare facility and stores the identification data and medication delivery data in the memory, wherein the processor also retrieves the vital signs data from the memory in response to a request therefor, formats the retrieved data in accordance with a format request, and displays the retrieved and formatted data on the display. The medication delivery data received by the processor comprises infusion delivery data for infusions in progress, the processor stores the delivery data for infusions in progress in the memory, and the processor also retrieves the delivery data for infusions in progress from the memory in response to a request therefor, formats the retrieved data in accordance with a format request, and displays the retrieved and formatted data on the display.

In yet other detailed aspects, the processor receives medication delivery data from a source through a wireless connection. One of the multiple data sources from which the processor receives medication delivery data is a portable data device. The processor receives medication delivery data from the portable data device through a wireless connection. The portable data device receives medication delivery data from an infusion pump and transfers that medication delivery data to the processor. The processor also retrieves patient identification data and medication delivery data from the memory and transmits the retrieved data to the portable data device. One of the multiple data sources from which the processor receives medication delivery data is a server.

In yet further aspects, the processor formats retrieved medication delivery data to display medication delivered to the patient organized by time. The processor formats retrieved medication delivery data to display medication delivered to the patient organized by at least one of hour, shift, and day. In another aspect, the processor formats retrieved medication delivery data to display medication delivered to the patient organized by unit of measure. The processor formats retrieved medication delivery data to display medication delivered to the patient organized by at least one of milligrams, and micrograms. The processor formats retrieved data in accordance with predetermined, selectable data formats. The processor formats retrieved data in accordance with an operator-created, non-preprogrammed data format.

In yet a further aspect, the processor compares future medication delivery data to past and present medication delivery data, compares such future medication data to a medication limits database, and provides an alert on the display if an inconsistency is indicated by the comparison.

In accordance with method aspects of the application, there is provided a method for monitoring medication delivery to a patient while the patient is in a healthcare facility, the method comprising receiving patient identification data and medication delivery data about the patient from multiple sources in the healthcare facility while the patient is in the healthcare facility, storing identification data and medication delivery data about the patient, retrieving the stored patient identification data and medication delivery data in response to a request therefore, formatting the retrieved data in accordance with a format request, and displaying the formatted identification data and medication delivery data about the patient

In yet a more detailed aspect, the total delivery or input of fluids to the patient is monitored, and in a further aspect, the total output of fluids by the patient is monitored and a net fluid total is provided.

These and other advantages of the invention will become apparent from the following more detailed description when taken in conjunction with the accompanying drawings of illustrative embodiments.

### BRIEF DESCRIPTION OF THE DRAWINGS

FIGURE 1 is a block diagram representation of a system for monitoring the total medication delivery to a patient in a healthcare facility incorporating principles of the present invention and illustrating details of the interconnections of the hardware elements;
FIG. 2 is an example of a patient identification bracelet having the patient's name in text and other information in optically-readable barcode form;
FIG. 3 is a drawing of a label affixed to a medication container wherein the label includes the medication in text form and other information about the mediation is included in optically-readable barcode form;
FIG. 4 shows a modular patient care unit in which a programming module controls various infusion modules, such as volumetric infusion pumps and syringe pumps;
FIGS. 5 and 6 show examples of formats for displays of the total medication delivery to a patient in a particular healthcare facility;
FIG. 7 is a view of another embodiment of a monitoring system for monitoring medication delivery in which the monitoring system is capable of monitoring a remote database of meditation delivery data shown in block diagram form; and
FIG. 8 is a drawing of still another embodiment of a system for monitoring the medication delivery to a patient in a healthcare facility in which a portable data storage unit is used to facilitate data transfer.

### DETAILED DESCRIPTION OF THE PREFERRED EMBODIMENTS

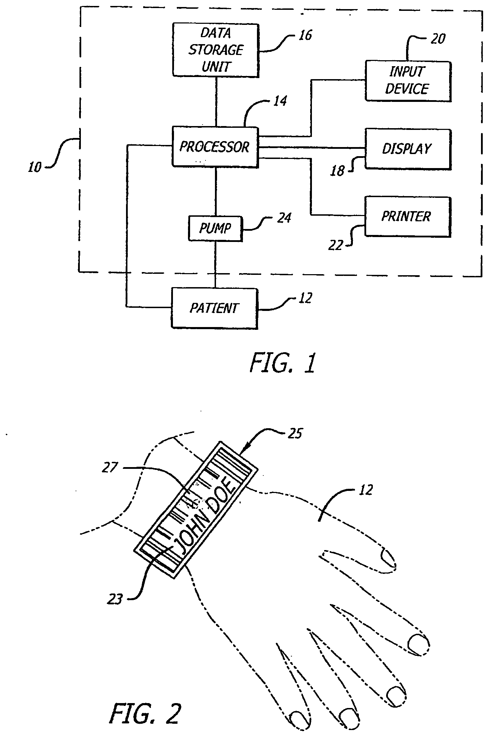

Referring now to the drawings in more detail in which like numerals used across several views indicate like or corresponding elements, there is shown in FIG. 1 a monitoring system 10 for monitoring the delivery of medication to a patient 12 while that patient is in a healthcare facility. The monitoring system includes a processor 14 for processing various operations of the monitoring system 10. Through an appropriate interface, the processor may be connected to a variety of peripheral equipment For example, FIG. 1 shows the processor operatively connected to a data storage unit 16 capable of storing data related to medication delivery, such as, for example, delivery status data, patient data pertaining to medical treatment, data input from a clinician, operator, or other user, medication delivery data, and other data needed to operate the monitoring system. The data storage unit may comprise a memory device of one type or another for storing data, well known to those skilled in the art. Further operatively connected to the processor is a display 18. During treatment of the patient, a clinician may utilize the display to visually observe any data related to the medication delivery to the patient, as well as other data relevant to the operation of the monitoring system. The display may take many forms well known to those skilled in the art. For example, an LCD or CRT display may be used, as well as others.

The monitoring system 10 also includes an input device 20 for allowing a clinician to access the monitoring system. Such an input device 20 may be a keypad, keyboard, mouse, touch pad, touch screen, or other means of entering data so that a clinician may input patient treatment data or specific commands for instructing the monitoring system. The input device may be stationary, located always at the processor, display, or other equipment, or may be mobile with a wireless or wired connection to the processor 14. Examples include personal digital assistants ("PDAs"), laptop computers or other mobile computers, stationary computers, multi-function cellular telephones, and other devices. A printer 22 may also be included in the monitoring system 10 for generating hard copy reports also relating to the total patient medication input. The monitoring system 10 may also include an infusion pump 24 as a means for delivering medication to the patient in a predetermined, controlled manner, or other medical instrument or device involved in the care or treatment of a patient. Multiple instruments or devices may be involved, such as multiple infusion pumps infusing multiple medical fluids into a patient at the same time, as an example.

Referring now to FIG. 2, a patient 12 entering a healthcare facility is usually provided with an identification device, such as a wristband, necklace, ankle band, or similar device 25 that is affixed to the patient 12 in a manner so that the patient can be identified even if the patient is unconscious or otherwise unresponsive. The identification device 25 may include patient data such as the patient's name, age, allergies, or other vital data relevant to the condition of the patient. In some cases, the identification device may include medication administration data about the patient such that the identification device may be thought of as providing a medication administration record ("MAR") of the patient. In the case of FIG. 2, the patient identification device not only includes the patient's name in human-readable text 23, but also stores patient data in an optically readable barcode 27. While barcodes can provide a large amount of data about a patient, they are difficult to update and in most cases must simply be replaced with a new barcode when data about the patient changes, such as newly-discovered allergies, or the delivery of additional medication to the patient. Replacing barcodes is an inconvenient activity. Other identification devices may be used, such as an RF identification ("RFID") device that is much easier to update with patient data. Nevertheless, a patient's name and other information, such as an identification number assigned by the healthcare facility, may be printed in human readable form on such identification device as a safety precaution. It is expected that in the future that other types of data storage devices will become available that allow for easier update.

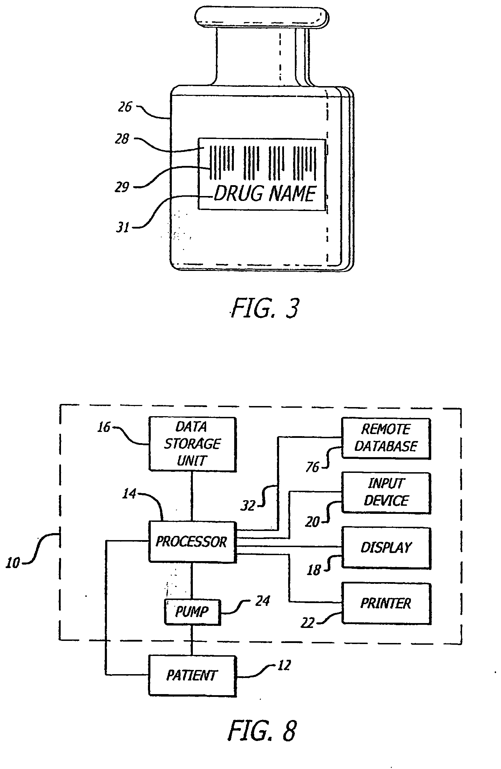

After the patient 12 is admitted to the healthcare facility, the patient is typically examined, evaluated by a physician, and a course of treatment or care is prescribed. The physician prescribes the course of treatment by preparing an order which may request an administration of a particular medication to the patient. Typically, the request is made by filling in a form or writing the order on a slip of paper to be submitted to the hospital's pharmacy. Once the order is received by the pharmacy, it is evaluated and processed. The pharmacy then prepares the medication according to the requirements of the physician and packages it in a container 26, such as that shown in FIG. 3. Medication delivery data such as, for example, the patient's name, the medication name, and the appropriate medication delivery parameters, such as dosage and period of administration may be represented on a label 28 that is then affixed to the medication container.

As in the last case, a barcode 29 is used in FIG. 3 to supply certain data. Also in this case, the name of the medication 31 is legibly printed in text along with the barcode, for safety purposes. The barcode may contain the name of the medication as well as other information, such as the name of the patient, and particulars about the medication. The barcode may also contain additional data, such as the delivery parameters for the medication. Such delivery parameters may include pump operating parameters such as, as an example, flow rate for a continuous infusion and volume to be infused, or may include multiple flow rates where infusions at different rates over certain time periods are to be administered.

Generally, the medication is delivered to the appropriate care-giving unit where a nurse administers the medication to the appropriate patient at the scheduled time. Referring again to FIGS. 1-3, prior to the administration of the medication to the patient 12, the patient is identified to the monitoring system 10. Via the display 18, the monitoring system prompts the nurse to enter certain patient data such as that found on the patient identification device 25 or other vital data related to the condition of the patient. As the data is input via the input device 20, it is analyzed by the processor 14, and then stored in the data storage unit 16. The data storage unit houses the patient data for as long as the patient is treated by the healthcare facility. As a result, the monitoring system is able to locate and display all IV infusion profiles and medication deliveries conducted by the monitoring system to the particular patient 12. This is helpful in keeping accurate records.

Once the patient is identified, the monitoring system 10 will perform a verification process before allowing the medication to be administered. The monitoring system prompts the nurse to enter medication delivery data regarding the medication to be delivered and the appropriate medication delivery parameters such as those found on the label 28 of the medication container 26. Such data may be entered manually by the nurse, or in the case of machine-readable data, such as a barcode, the data may be scanned in by use of a barcode reader or an RFID reader or interrogator, depending on the data storage device or devices used. For certain medications, the nurse may be prompted to enter data descriptive of a selected patient parameter or parameters, such as a current vital sign. For example, the nurse may be prompted to measure and enter a value for the patient's blood pressure before administering the medication.

Where the medication is to be delivered using, for example, an infusion pump 24, the nurse first inputs into the monitoring system 10 the appropriate configuration parameters for the infusion. Such parameters may be found on the label 28 that is affixed to the medication container 26 and can be read in with a barcode reader (not shown) such as by scanning the barcode. Alternatively, the appropriate configuration parameters may already be stored in the data storage unit 16. In such a case, the nurse may access the data storage unit via the input device 20 and view the parameters on the display 18. Upon visual verification, the nurse may set the parameters to configure the pump wherein the parameters are communicated from the data storage unit to the pump. In another approach, the nurse may manually enter the parameters at the pump using a keypad at the pump or by other means. Once the infusion pump is configured, the nurse may start the infusion either by pressing an appropriate control on the infusion pump or inputting a command in the input device. Upon start of the infusion, the pump transmits a signal to the processor 14 marking the start of the infusion. The processor then logs this information into the data storage unit. Information may be logged continually, such as rate changes, end of infusion, and other information.

The monitoring system 10 may also include a drug library database stored in the data storage unit 16 having ranges of acceptable values for various operating parameters of the infusion pump 24 and/or dosages of the medication. The drug library database would be accessible by the processor 14. Additionally, the drug library database may include various data sets of acceptable operating parameters linked to the particular medication being delivered. For example, data sets for NICU, CCU, General Surgery, and others. In this embodiment, the processor compares the operating parameter values for the particular medication to the medication database range of acceptable values. If the entered value for the operating parameter is within the range of accepted values and/or the dosage of the medication is within accepted values, the processor will permit the parameter to be entered into the infusion pump and the infusion started. If not, an alert is provided to the operator. The nurse is then prompted to correct the discrepancy by entering the correct data. In the event the nurse determines that the discrepancy cannot be corrected but that the discrepancy is minor and will not affect the accuracy or safety of the delivery of the medication, the nurse may override the alert. However, if the discrepancy is not minor and cannot be overridden, the nurse may not give the medication to the patient at these parameters because the processor will not permit entry of the offending operating parameter or parameters into the pump and a reevaluation of the medication treatment will be necessary.

In the case of orally-administered medication, such as pills, or other form of delivery of medication, the clinician likewise indicates to the monitoring system 10, through the processor 14 or by other means, the same identification of medication, medication particulars such as dosage, and any other information required by the system. These may be entered manually or through a scanning process, such as a barcode or RFID, or other wired or wireless data transfer process. The monitoring system may also check these medications against a drug library to identify any irregularities in the medication to be administered and indicate alarms if any irregularity is found.

The processor 14 verifies that the right medication is being given to the right patient 12 in the right dose and at the right time by evaluating the known patient data versus the known medication delivery data. Additionally, the identity of the nurse dispensing the medication may be required by the processor and entered into the input device 20 by the nurse before any administration may take place. This permits verification that an authorized person is administering the medication. The nurse's identity is recorded in the data storage unit 16 for possible future reference. Entry of the nurse's data may also be performed as described above. It may be entered manually or in the case of machine-readable nurse data, such as that contained in a personal badge having a barcode, RFID device, or other, it may be scanned in through an appropriate reader, or other wired or wireless data transfer process.

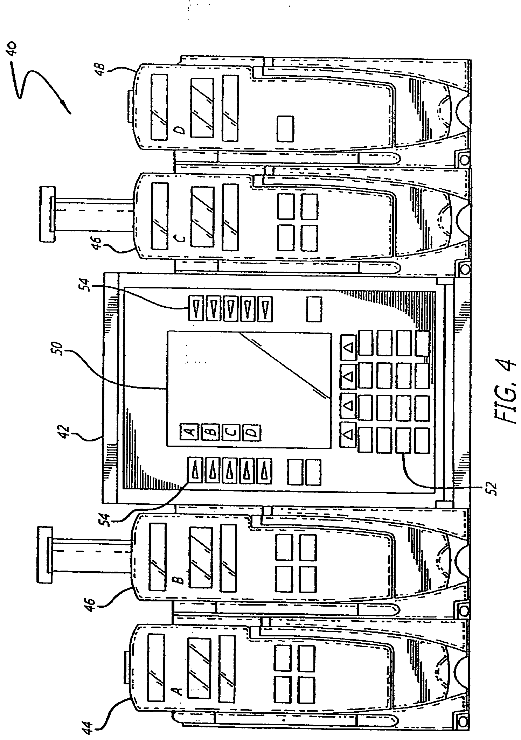

The pump, processor, data storage unit, data input device, and display may take different forms. In one embodiment, a module controller, or controller, includes a processor, memory, communications devices, and other associated equipment. Referring now to FIG. 4, a view of one configuration of such a patient point-of-care system 40 is shown. In this patient care unit, a controller 42 is used to control a volumetric infusion pump module 44 designated as channel "A" as well as two syringe pump modules 46 designated as channels "B" and "C," and another volumetric infusion pump module as channel "D." The controller includes a display 50 suitable for displaying total patient medication data, as well as an input device, in this case a keypad 52. The keypad comprises "hard" keys, i.e., keys that have a dedicated use, and "soft" keys located alongside the display. The soft keys are programmed by the processor and their programmed function is indicated by the display at a location adjacent the key. Such key types and their programming are well known to those skilled in the art and no further information is provided here. Although shown as separate devices in FIG. 4, the pump and controller may be located in the same housing in another embodiment. Further in the case of FIG. 4, the controller is in communication with a local area network or wide area network to receive data about a patient and patient medication deliveries from other data sources. Such communication with the network or with other devices such as a server or servers, may be either wired or wireless.

In the above embodiment of FIG. 4, the controller 42 may satisfy the requirements of the processor 14 of FIG. 1, the interconnected pumping modules 44, 46 and 48 may satisfy the requirements of the pump 24 of FIG. 1, the keypads 52 and 54 may satisfy, at least partially, the requirements of the input device 20 of FIG. 1, the display 50 may satisfy the requirements of the display 18 of FIG. 1, and an internal memory (not shown) of the controller 42 may satisfy, at least partially, the requirements of the data storage unit 16 of FIG. 1.

Referring again to FIG. 1, the monitoring system 10, of which the pump 24 forms a part in this embodiment, monitors the infusion process in a real-time manner, providing alerts on the display 18 and allowing intervention by nurses if necessary. The processor 14, suitably programmed, controls the monitoring system to automatically record the start time of the infusion, query the pump 24 periodically throughout the infusion, maintain a continuous log of the infusion, and record the end time of the infusion and the volume infused in the data storage unit 16. Other data such as the name of the medication, the amount of medication delivered, and other important data concerning the progress and particulars of the infusion may be recorded in the data storage unit. As a result, manual record keeping of such data on a paper chart is made unnecessary. Furthermore, all data, or a selected portion of the data, may be shown on the display 18 throughout the infusion to provide a visual presentation of the status of the infusion.

In a further aspect in accordance with the invention, the processor 14 is also programmed to format the medication delivery data related to the patient 12 in a plurality of selectable formats according to instructions received from the input device 20 for presentation on the display 18. As used herein, "format" is used in its broadest sense; that is, "format" refers to any method of arranging information that is to be stored or displayed. In one embodiment, preprogrammed formats may be selected by the operator of the display. For example, the processor is preprogrammed to display total medication delivered in a format based on time. In such case, the display may be formatted by total medication delivered by every hour, every shift, or every day. Such data may also be formatted by unit of time in other ways, such as volume infused per clinician work shift. The nurse or other operator also may use the system to display amounts of medication delivered during a specific time frame in the past. This data is helpful in monitoring certain medications that cannot be given within a predetermined time frame of administration of another medication. In another embodiment, the processor is programmed to display total medication delivered organized by unit of measure. In one example, the display may be organized or formatted by micrograms, milligrams or by grams. The data may be formatted to be organized by specific medication in other ways. In a further embodiment, the processor is also programmed to permit an operator such as a nurse to create a non-preprogrammed data format with the input device. Patient medication delivery data may then be presented in any format as programmed by the operator into the processor.

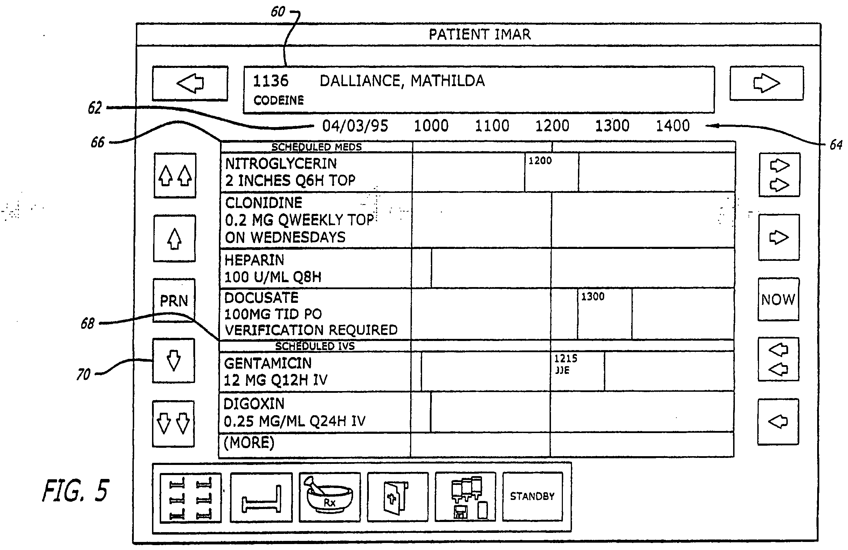

As an example of the type of formats of total patient medication, reference is now made to FIG. 5. Data concerning the patient, who is identified both by her name in text and by a numeric identifier 60, is presented. In this case, the operator of the data presentation has chosen 04/03/95 as the date 62 of interest. A block of certain hours of the day 64 (1000 through 1400) is presented and for those hours, a list of SCHEDULED MEDS 66 and a list of SCHEDULED IVS 68 are presented. In this case, more SCHEDULED IVS exist as indicated by the word (MORE) in the last row and a "page down" type of command in the form of a downwardly pointing arrow 70 may be selected to see more information. The screen contains many navigation arrows to move among different information that can be presented. The approach of using navigation arrows is well known to those skilled in the art and no further information about their use or implementation is presented here.

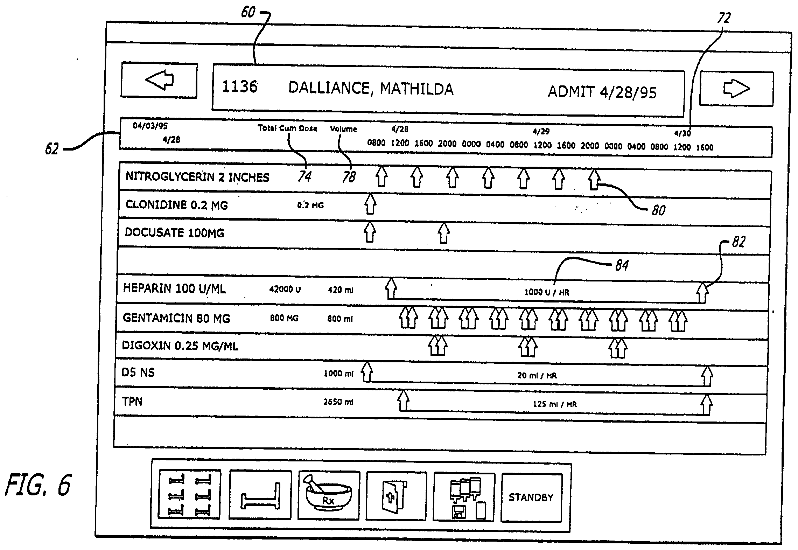

FIG. 6 provides a more detailed display of medication information about the same patient. As before, the patient is indicated by text and numeric identifier 60 at the top of the display. However in this case, a view of three days 72 is presented; i.e., 4/28, 4/29, and 4/30. A column of "Total Cum Dose" 74 (total cumulative dose) is provided as is a column for "Volume" 78. The delivery times are indicated by arrows 80 and the infusion periods are indicated by arrows interconnected by horizontal lines 82. Infusion rates 84 are also provided.

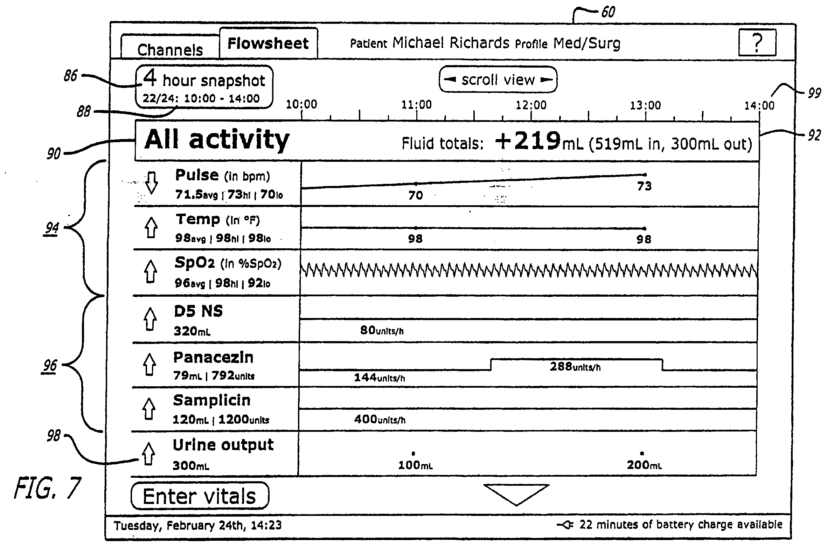

FIG. 7 provides yet another display of medication data formatted in a different way. In this display, the patient is identified 60 at the top of the display. The date 86 of the data and the hours 88 of the data are indicated at the upper left. In this case, "All activity" 90 was selected as the data to be presented. The Fluid totals are presented 92 with a positive or negative sign to indicate an increase or decrease in fluids of the patient respectively. Vital signs 94 are provided, including SpO₂. All fluids going into the patient 96 and Urine output 98 are presented. The hours 99 of input and output of fluids are shown.

The above features permit a nurse to select any format of data pertaining to a patient's total medication delivery for the entire stay at the healthcare facility and evaluate that data in view of a past, present, or future medication delivery. Referring also to FIG. 1 again, the monitoring system 10 in accordance with the invention eliminates the need for the nurse to rely on memory or a written paper chart to assess medication delivery. Moreover, the monitoring system may assist the nurse in efficiently delivering care to the patient by providing the ability to perform a plurality of queries of the patient's past, present, and future medication administration.

The monitoring system 10 is also connected to a printer 22 to enable a nurse or other operator to create a written record of the total medication deliveries to the patient 12. Because of the versatility in formatting the data about a patient's total medication delivery, the nurse may create various displays or printed reports for use in ensuring proper medication of the patient. The monitoring system 10 may also be programmed to operate automatically to display and/or print reports of a standard format at predetermined intervals as determined by the needs of the particular nurse or healthcare facility. For example, predetermined displays of total patient medication delivery data may be displayed at times such as at the end of an infusion and printed.

In conjunction with the processor 14 being programmed to display all medication delivery data for the patient 12 while in the healthcare facility obtained from the infusion pump 24 with which it is associated, the processor is also programmed to retrieve medication delivery data related to the patient from other memory devices in the healthcare facility in accordance with another aspect of the invention. For example, if the processor is a part of the infusion pump 24 or is in communication with that pump such as is shown in FIG. 4, it will receive data from the infusion pump with which it is associated. However, other infusion pumps may have been used on the patient at other times in the healthcare facility. The present processor 14 and perhaps the present nursing staff may not be aware of these other medication deliveries to the patient 12. The pharmacy may have further information about medication delivered to the patient, especially in the case where medication was prescribed but an infusion pump was not used for delivery. In both examples, it would he helpful to the nursing staff to see the total medication delivery at the display of the present pump before activating that pump for further medication delivery. In accordance with this aspect of the invention, the processor 14 may access other databases of medication delivery for this patient through a local area network, a wide area network, or other communication network with which it is associated. Other mediation delivery data generated by other devices will be obtained and placed in the data storage unit 16 for use by the operator or operators of the infusion pump 24 associated with this processor 14.

In further detail, the monitoring system 10 may be configured to access a remote database 76, as shown in FIG. 8, for acquiring data about a particular patient 12. The remote database may be one of a plurality of data sources that serve as a storage unit for data generated in the healthcare facility regarding the care provided to the patient 12 collected by means other than the pump 24 that the patient is currently connected with. The remote database 76 may contain data related to medications delivered to the same patient 12. It may further contain other data related to the condition of the patient. This remote database 76 may be data residing in the memory of another pump, a pharmacy database, or other storage device for data concerning the patient 12. The remote database 76 is included within the block labeled numeral 10 in FIG. 7 because it is reachable by the processor 14 through a communication line, either wired or wireless. It therefore becomes part of the monitoring system 10. It can thus be seen that pumps and/or processors that are networked together can share data about the patient 12 thus permitting a nurse or other professional to evaluate the total patient input or medication while at the healthcare facility.

The process of gathering medication delivery data about a patient 12 may be automated. For example, as soon as a patient is associated with a pump 24, the processor 14 also associated with that pump may request medication delivery data from all other devices on the network that the processor is on. This medication delivery data may then be stored in the data storage unit 16 associated with that processor and pump for use by the operator of that pump on the patient.

Thus, when deemed necessary by the processor 14 or a clinician to deliver proper treatment, the monitoring system 10 will access patient 12 medication data beyond what is found in its own data storage unit 16, format that data as requested by the clinician, and evaluate the data retrieved from remote databases. Evaluation by the processor 14 may occur in accordance with a drug library-type database and program where infusion parameters are compared to acceptable limits in the drug library-type database. For example, an infusion rate for a particular drug is compared against such acceptable limits in the drug library and if the programmed infusion rate is higher than the limit in the drug library, an alarm is provided. As another example, allergies of the patient are compared to information in the database and if a drug is not suitable for a patient with such allergies, an alarm will be provided should a clinician attempt to infuse such drug. By means such as a local area network (LAN) cable 32, or a wide area network, the monitoring system 10 may collect any necessary patient medication history information from the remote database 76 thus eliminating the need for the clinician to manually input such data into the monitoring system 10. The network with which the pumps or processors are associated may be wired or wireless.

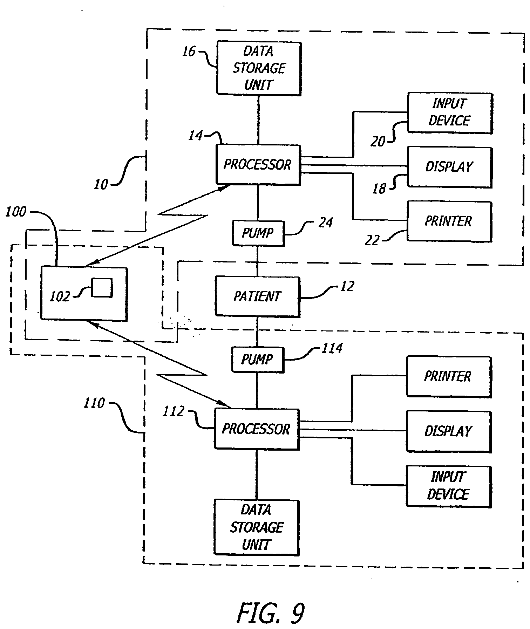

In the case where no network exists in a healthcare facility, the monitoring system 10, as shown in FIG. 9, may include a portable data storage unit 100 that may be manually moved to patient sites to collect and distribute patient-specific medication delivery data. The portable data storage unit may take the form of a personal digital assistant ("PDA"), tablet computer, laptop computer, or other device having adequate memory, processing power, and communications abilities. The portable data storage unit may be operatively connected to the processor 14 through wired or wireless connection. The embodiment of FIG. 9 shows a wireless connection. Much like the data storage unit 16, the portable data storage unit 100 is capable of collecting a variety of information including patient data input from a user, medication delivery data, and other data, and storing it in a local memory 102. As the monitoring system monitors the medication delivery, the portable data storage unit records all information pertaining to any medication delivery being performed into its memory which can be a portable read/write medium such as a fixed disk, CD, DVD, floppy disk, non-volatile flash memory, or other suitable storage medium. The storage medium 102 may be removed from the portable data storage unit to operate in other similar devices. The data recorded and transmitted may include all data related to medication delivery of each patient.

Not only is the portable data storage unit 100 capable of receiving and storing data, it will also respond to requests from monitoring systems for data concerning a patient that a monitoring system processor may be serving at the time. It will transmit that data to the requesting monitoring system. Thus, the portable data storage unit effectively acts as a network that is available when physically present near the processor of interest.

This is shown in block diagram form in FIG. 9 in that the portable data storage unit 100 has become a member of two monitoring systems 10 and 110 and has a wireless connection with both. In the embodiment shown, the processor 14 of the first monitoring system 10 is wirelessly connected to the portable data storage unit 80 as is the processor 112 of the second monitoring system 110. It will be noted that an infusion pump 24 of the first monitoring system is connected with the patient 12 and an infusion pump 114 of the second monitoring system 110 is also connected to the same patient 12. The monitoring systems are shown as overlapping the portable data storage unit 100. Although shown together in this figure and apparently infusing fluid to the same patient 12 at the same time, the connections with the portable data storage unit 100 may actually be separated in time. They are shown together in FIG. 9 to more clearly display the effective operation of a mobile portable data storage unit that contains the data of the same patient and how that data can be made available to different monitoring systems throughout the healthcare facility.

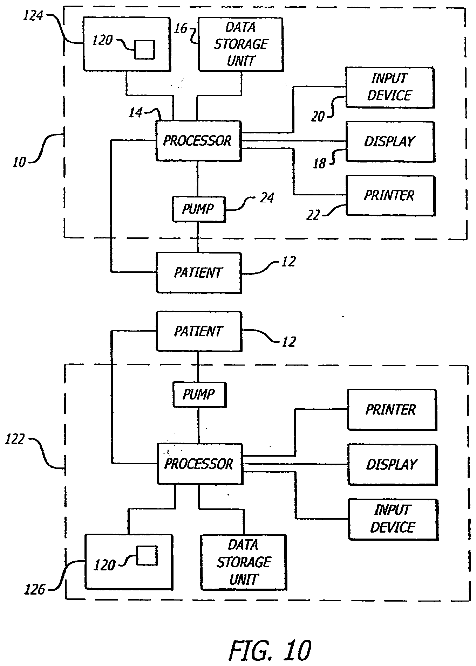

Referring now to FIG. 10, a slightly different embodiment is shown where a non-volatile memory device 120 is transported among patient care units or monitoring units. In this figure, the same patient 12 is shown as undergoing treatment with a first monitoring system 10 and is taken off the first system 10 and subsequently attached to a second monitoring system 122. In this embodiment, the second monitoring system 122 learns of the medication deliveries administered to the patient by the first monitoring system 10 by reading the first system's read/write medium 120 once it is removed from the first system's 10 portable data storage unit 124 and mounted in the second system's portable data storage unit 126. Thus, a clinician responsible for the second monitoring system would not have to analyze a written chart detailing the first system's delivery profile. Moreover, the clinician would not need to input the first monitoring system's 10 information into the second monitoring system 122 in order for the second monitoring system to be able to comprehensively display all medication deliveries to this patient 12. Thus, the transfer of information is made easier, more accurate, and more reliable. The above may arise in the situation where multiple infusion pumps exist throughout a healthcare facility.

In another embodiment, the first monitoring system 10 and the second monitoring system 122 may be connected to one another, either through wired or wireless direct connection, or through an intermediate data transfer apparatus, such as a server, the Internet, a local area network (LAN), a wide area network (WAN), Ethernet, or other, allowing the transfer of data from one system to the other.

Although the invention has been described in terms of preferred structures, it will be apparent to one skilled in the art that obvious modifications may be made without departing from the invention. It is intended that all such modifications are included in the scope of the invention as defined herein and protected by the appended claims.

## Claims

1. A system for monitoring medication delivery to a patient while the patient is in a healthcare facility, the system comprising:
one or more infusion pumps (24, 44, 46, 48); and
a controller (14, 42) configured to be coupled to the one or more infusion pumps, the controller comprising:
a memory (16) in which is stored identification data about the patient, medication delivery data about the patient, data for infusions in progress, and vital signs data about the patient; and
a display (18, 50) configured to display the identification data, medication delivery data, and vital signs data about the patient,
wherein the controller (14, 42) is configured to:
accept input parameters, by way of a scanning of a barcode or radio frequency identification (RFID) device, including a medication and a flow rate for administration of the medication and an indication to start an infusion of the medication;
receive, from a remote database (76) via a network, patient identification data, and medication delivery data, and patient vital signs data about the patient,
**characterized in that** the controller is further configured to:
receive the patient identification data and medication delivery data from multiple sources in the healthcare facility while the patient is in the healthcare facility, including receiving the medication delivery data from a different infusion pump used to administer a medication to the same patient at other times in the healthcare facility, and store the identification data and medication delivery data in the memory;
verify, based on the patient identification data, medication delivery data, patient vital signs data, and the input parameters, that the right medication is being given to the right patient in the right dose and at the right time before allowing the medication to be administered, wherein an entered value for an input parameter is compared to an acceptable value or range received from the remote database and if the entered value is within the acceptable value or range then the input parameter will be entered and the medication allowed to be administered, otherwise an alert is provided by the controller; and
accept a request to display a portion of the medication delivery data, whereupon the controller (14, 42) retrieves the portion of the medication delivery data from the memory, and provides for display on the display (18, 50) multiple graphics organized by time, including respective fluid delivery rates over time for multiple medications administered to the same patient, and respective vital sign measurements over time for multiple vital signs for the same patient, based on the medication delivery data.

2. The system of claim 1 wherein one of multiple data sources from which the controller (14, 42) receives the medication delivery data is a portable data device.

3. The system of claim 2 wherein the controller (14, 42) is further configured to receive the medication delivery data from the portable data device through a wireless connection.

4. The system of claim 2 wherein the portable data device is configured to receive the medication delivery data from the different infusion pump and to transfer the medication delivery data to the controller (14, 42).

5. The system of claim 2 wherein the controller (14, 42) also is further configured to retrieve stored patient identification data and stored medication delivery data from the memory and transmit the stored patient identification data and the stored medication delivery data to the portable data device.

6. The system of claim 1 wherein one of multiple data sources from which the controller (14, 42) receives the medication delivery data is a server remote from the controller.

7. The system of claim 1 wherein the controller (14, 42) is further configured to format the retrieved medication delivery data to display medication delivered to the patient organized by unit of measure.

## Patentansprüche

1. System zur Überwachung einer Medikamentenverabreichung an einen Patienten, während sich der Patient in einer Gesundheitseinrichtung befindet, wobei das System aufweist:
eine oder mehrere Infusionspumpen (24, 44, 46, 48); und
eine Steuerung (14, 42), die dazu ausgelegt ist, mit der einen oder den mehreren Infusionspumpen gekoppelt zu werden, wobei die Steuerung Folgendes umfasst:
einen Speicher (16), in dem Identifikationsdaten über den Patienten, Medikamentenverabreichungsdaten über den Patienten, Daten über laufende Infusionen und Vitalzeichendaten über den Patienten gespeichert werden; und
einen Monitor (18, 50), der dazu ausgelegt ist, die Identifikationsdaten, Medikamentenverabreichungsdaten und Vitalzeichendaten über den Patienten anzuzeigen,
wobei die Steuerung (14, 42) ausgelegt ist zum:
Annehmen von Eingangsparametern durch Scannen eines Strichcodes oder eine Hochfrequenz-Identifikationsvorrichtung (RFID), einschließlich eines Medikaments und einer Durchlaufmenge für die Verabreichung des Medikaments und einer Angabe zum Starten einer Infusion des Medikaments;
Empfangen, von einer entfernten Datenbank (76) über ein Netzwerk, von Patientenidentifikationsdaten, Medikamentenverabreichungsdaten und Patienten-Vitalzeichendaten über den Patienten,
**dadurch gekennzeichnet, dass** die Steuerung darüber hinaus ausgelegt ist zum:
Empfangen der Patientenidentifikationsdaten und Medikamentenverabreichungsdaten von mehreren Quellen in der Gesundheitseinrichtung, während sich der Patient in der Gesundheitseinrichtung befindet, einschließlich des Empfangens der Medikamentenverabreichungsdaten von einer anderen Infusionspumpe, die zur Verabreichung eines Medikaments an denselben Patienten zu anderen Zeiten in der Gesundheitseinrichtung verwendet wird, und Speichern der Identifikationsdaten und Medikamentenverabreichungsdaten im Speicher;
Überprüfen, beruhend auf den Patientenidentifikationsdaten, Medikamentenverabreichungsdaten, Patienten-Vitalzeichendaten und den Eingangsparametern, ob dem richtigen Patienten das richtige Medikament in der richtigen Dosis und zum richtigen Zeitpunkt gegeben wird, bevor die Verabreichung des Medikaments erlaubt wird, wobei ein eingegebener Wert für einen Eingangsparameter mit einem zulässigen Wert oder Bereich verglichen wird, der von der entfernten Datenbank empfangen wird, und, wenn der eingegebene Wert innerhalb des zulässigen Werts oder Bereichs liegt, der Eingangsparameter dann eingegeben und die Verabreichung des Medikaments erlaubt wird, andernfalls von der Steuerung eine Warnung ausgegeben wird; und
Annehmen einer Aufforderung zur Anzeige eines Teils der Medikamentenverabreichungsdaten, woraufhin die Steuerung (14, 42) den Teil der Medikamentenverabreichungsdaten aus dem Speicher abruft und zur Anzeige auf dem Monitor (18, 50) mehrere nach Zeit geordnete Grafiken bereitstellt, einschließlich der jeweiligen Fluidzufuhrraten im Zeitverlauf für mehrere Medikamente, die demselben Patienten verabreicht wurden, und jeweiliger Vitalzeichenmessungen im Zeitverlauf für mehrere Vitalzeichen für denselben Patienten, basierend auf den Medikamentenverabreichungsdaten.

2. System nach Anspruch 1, wobei eine von mehreren Datenquellen, von denen die Steuerung (14, 42) die Medikamentenverabreichungsdaten empfängt, ein tragbares Datengerät ist.

3. System nach Anspruch 2, wobei die Steuerung (14, 42) ferner dazu ausgelegt ist, die Medikamentenverabreichungsdaten von dem tragbaren Datengerät über eine drahtlose Verbindung zu empfangen.

4. System nach Anspruch 2, wobei das tragbare Datengerät dazu ausgelegt ist, die Medikamentenverabreichungsdaten von der anderen Infusionspumpe zu empfangen und die Medikamentenverabreichungsdaten an die Steuerung (14, 42) zu übertragen.

5. System nach Anspruch 2, wobei die Steuerung (14, 42) darüber hinaus dazu ausgelegt ist, gespeicherte Patientenidentifikationsdaten und gespeicherte Medikamentenverabreichungsdaten aus dem Speicher abzurufen und die gespeicherten Patientenidentifikationsdaten und die gespeicherten Medikamentenverabreichungsdaten an das tragbare Datengerät zu übertragen.

6. System nach Anspruch 1, wobei eine von mehreren Datenquellen, von denen die Steuerung (14, 42) die Medikamentenverabreichungsdaten empfängt, ein von der Steuerung entfernter Server ist.

7. System nach Anspruch 1, wobei die Steuerung (14, 42) ferner dazu ausgelegt ist, die abgerufenen Medikamentenverabreichungsdaten zu formatieren, um ein dem Patienten verabreichtes Medikamente nach Maßeinheit geordnet anzuzeigen.

## Revendications

1. Système de surveillance de fourniture de médicament à un patient tandis que le patient se trouve dans un établissement de soins de santé, le système comprenant :
une ou plusieurs pompes à perfusion (24, 44, 46, 48) ; et
un dispositif de commande (14, 42) configuré pour être couplé à l'une ou aux plusieurs pompes à perfusion, le dispositif de commande comprenant :
une mémoire (16) dans laquelle sont stockées des données d'identification relatives au patient, des données de fourniture de médicament relatives au patient, des données pour des perfusions en cours, et des données de signes vitaux relatives au patient ; et
un affichage (18, 50) configuré pour afficher les données d'identification, les données de fourniture de médicament, et les données de signes vitaux relatives au patient,
sachant que le dispositif de commande (14, 42) est configuré pour :
accepter des paramètres d'entrée, moyennant un balayage d'un code à barres ou d'un dispositif d'identification par radiofréquence (RFID), incluant un médicament et un débit d'administration du médicament et une indication de démarrer une perfusion du médicament ;
recevoir, depuis une base de données (76) distante via un réseau, des données d'identification de patient, et des données de fourniture de médicament, et des données de signaux vitaux de patient relatives au patient,
**caractérisé en ce que** le dispositif de commande est en outre configuré pour :
recevoir les données d'identification de patient et les données de fourniture de médicament depuis de multiples sources dans l'établissement de soins de santé tandis que le patient se trouve dans l'établissement de soins de santé, incluant la réception des données de fourniture de médicament depuis une pompe à perfusion différente utilisée pour administrer un médicament au même patient à d'autres moments dans l'établissement de soins de santé, et stocker les données d'identification et les données de fourniture de médicament dans la mémoire ;
vérifier, sur la base des données d'identification de patient, des données de fourniture de médicament, des données de signes vitaux de patient, et des paramètres d'entrée, que le bon médicament est donné au bon patient dans la bonne dose et au bon moment avant d'autoriser l'administration du médicament, sachant qu'une valeur saisie pour un paramètre d'entrée est comparée à une valeur ou une plage admissible reçue depuis la base de données distante et, si la valeur saisie est comprise dans la valeur ou la plage admissible, le paramètre d'entrée sera saisi et l'administration du médicament sera autorisée ; sinon, une alerte est émise par le dispositif de commande ; et
accepter une demande d'afficher une partie des données de fourniture de médicament, suite à quoi le dispositif de commande (14, 42) récupère la partie des données de fourniture de médicament depuis la mémoire, et fournit pour affichage sur l'affichage (18, 50) de multiples graphiques organisés chronologiquement, incluant des débits de fourniture de fluide respectifs dans le temps pour de multiples médicaments administrés au même patient, et des mesures de signes vitaux respectifs dans le temps pour de multiples signes vitaux pour le même patient, sur la base des données de fourniture de médicament.

2. Le système de la revendication 1, sachant qu'une de multiples sources de données depuis lesquelles le dispositif de commande (14, 42) reçoit les données de fourniture de médicament est un dispositif de données portatif.

3. Le système de la revendication 2, sachant que le dispositif de commande (14, 42) est en outre configuré pour recevoir les données de fourniture de médicament depuis le dispositif de données portatif via une connexion sans fil.

4. Le système de la revendication 2, sachant que le dispositif de données portatif est configuré pour recevoir les données de fourniture de médicament depuis la pompe à perfusion différente et pour transférer les données de fourniture de médicament au dispositif de commande (14, 42).

5. Le système de la revendication 2, sachant que le dispositif de commande (14, 42) est en outre configuré pour récupérer des données d'identification de patient stockées et des données de fourniture de médicament stockées depuis la mémoire et transmettre les données d'identification de patient stockées et les données de fourniture de médicament stockées au dispositif de données portatif.

6. Le système de la revendication 1, sachant qu'une de multiples sources de données depuis lesquelles le dispositif de commande (14, 42) reçoit les données de fourniture de médicament est un serveur distant du dispositif de commande.

7. Le système de la revendication 1, sachant que le dispositif de commande (14, 42) est en outre configuré pour formater les données de fourniture de médicament récupérées pour afficher un médicament fourni au patient de manière organisée par unité de mesure.
